# EUROPEAN PATENT APPLICATION

(11) **EP 1 639 902 A1**
(43) Date of publication of application: **29.03.2006**
(21) Application number: 04733098.0
(22) Date of filing: 14.05.2004
(51) Int. Cl.: A23L 1/30, A61K 31/352, A61P 3/00, A61P 43/00

(54) **MOVEMENT PHYSIOLOGY IMPROVER**

(30) Priority: 19.06.2003 JP 2003174542
(71) Applicant: SUNTORY LIMITED, Osaka-shi, Osaka 530-8203 (JP)
(72) Inventor: IINO, Taeko, 5200113 (JP); TANAKA, Hiroaki, Fukuoka-shi, Fukuoka;8140155 (JP); SAWAKI, Keisuke, 2750013 (JP); KOIKAWA, Natsue, 2850862 (JP); KISO, Yoshinobu, 5670872 (JP)
(74) Representative: Vossius & Partner
(86) International application number: PCT/JP2004/006548
(87) International publication number: WO 2004/112510

(57) **Abstract**

The present invention provides an agent for improving physiological motor functions which is **characterized in** containing proanthocyanidin as an effective ingredient. It is useful as an agent for preventing and improving the fatigue in which rise in lactic cid value upon physical exercise is suppressed whereby muscular fatigue is reduced and continuous exercise is made easy, and particularly as an agent for prevention and improvement of muscular fatigue, and as a health food for prevention and improvement of fatigue.

## Description

### TECHNICAL FIELD

The present invention relates to a composition containing proanthocyanidin as an effective ingredient and acts on improvement of physiological motor functions or, in other words, to an agent for improving physiological motor functions. More particularly, the present invention relates to an agent for preventing and improving the fatigue in which increase of lactic acid value upon physical exercise is suppressed whereby muscular fatigue is reduced and continuous exercise is made easy or particularly to an agent for prevention and improvement of muscular fatigue, and to a health food for prevention and improvement of fatigue.

### BACKGROUND ART

Proanthocyanidin is a kind of polyphenols contained in plants and has been known to have various activities such as antioxidant action (Non-Patent Document 1 and Non-Patent Document 2). With regard to the action of proanthocyanidin, an activity of elimination of superoxide radicals has been reported and its synergistic action with vitamin C having an antioxidant action has been reported (Non-Patent Document 1). In addition, with regard to a clinical action, its therapeutic effect for chronic pancreatitis has been known (Non-Patent Document 1 and Non-Patent Document 2).

(Non-Patent Document 1) Bagchi, D. et al., Toxicology, 2000, Volume 148, pages 187 to 197.

(Non-Patent Document 2) Fremont, L. et al., Life Sci., 1999, Volume 64, pages 2511 to 2521.

### DISCLOSURE OF THE INVENTION

### PROBLEMS TO BE SOLVED BY THE INVENTION

Together with an increase in orientation toward good health in recent years, there is a tendency regardless of aged and young or female and male to positively carry out physical exercise for the purpose of maintenance of health and development of one's physical strength. For example, it is recommended to aged people to conduct appropriate trainings to aim improvement of aerobic working ability and enhancement of muscle power for a purpose of prevention of being bedridden and promotion of good health. However, if physical exercise is started suddenly, there is a problem that disintegration of muscle cells and muscular fatigue take place as a result of excessive production of free radicals in muscle cells and accumulation of fatigue substances in muscle tissues.

In the case of athletes, an intensive training is conducted for a predetermined period for the purpose of enhancement of muscular power and stamina. However, when an excessive burden is applied to muscle tissues, that results in muscular fatigue and disintegration of muscle cells whereby continuance of physical exercise becomes difficult or unexpected trouble may happen. In order to make the physical exercise effective, it is important that burden to muscle cells and muscle tissues is reduced and that physical exercise is continuously carried out together with an appropriate load to the whole body. An object of the present invention is to provide a novel composition which reduces damage of muscle cells caused by physical exercise or, in other words, an agent for improving physiological motor functions and, more particularly, to provide an agent for prevention and improvement of fatigue, especially an agent for prevention and improvement of muscular fatigue, and a health food for prevention and improvement of fatigue.

### MEANS FOR SOLVING THE PROBLEMS

The present inventors have carried out intensive studies repeatedly for solving the above-mentioned problems with a presumption that suppression of peroxidation disorder of muscle cells eliminates the bad affection by physical exercise and also contributes in continuance of training and, as a result, they have confirmed that, when proanthocyanidin having a powerful antioxidant action is ingested, muscular damage in the initial stage of physical exercise and peroxidation disorder after ingestion for a long term are reduced whereupon conducting of a continuous physical exercise is made easy, and have achieved the present invention.

Thus, the present invention relates to the followings.
(1) An agent for improving physiological motor functions which is characterized in containing proanthocyanidin as an effective ingredient;
(2) The agent for improving physiological motor functions according to the above (1), wherein fatigue is prevented and improved;
(3) The agent for improving physiological motor functions according to the above (1), wherein muscular fatigue is prevented and improved;
(4) A health food for prevention and improvement of fatigue which is characterized in containing proanthocyanidin as an effective ingredient;
(5) The health food for prevention and improvement of fatigue according to the above (4), wherein the health food is a solid food, a gel-formed food or a beverage;
(6) The health food for prevention and improvement of fatigue according to the above (5), wherein the beverage is a refreshing beverage or a tea beverage;
(7) The agent for improving physiological motor functions according to the above (1), wherein the agent is used in the form of tablet, pill, capsule, granule, powder, diluted powders or liquid;
(8) The health food for prevention and improvement of fatigue according to the above (4), wherein the health food is used in the form of tablet, pill, capsule, granule, powder, diluted powder or liquid;
(9) The agent for improving physiological motor functions according to the above (1), wherein proanthocyanidin is an extract derived from pine bark;
(10) The agent for improving physiological motor functions according to the above (1), wherein proanthocyanidin is an oligomeric proanthocyanidin;
(11) The health food for prevention and improvement of fatigue according to the above (4), wherein proanthocyanidin is an extract derived from pine bark; and
(12) The health food for prevention and improvement of fatigue according to the above (4), wherein proanthocyanidin is an oligomeric proanthocyanidin.
   The present invention further relates to the followings.
(13)A method for improving physiological motor functions, characterized in that proanthocyanidin is administered to human being;
(14) The method for improving physiological motor functions according to the above (13), wherein fatigue is prevented and improved;
(15) The method for improving physiological motor functions according to the above (13), wherein muscular fatigue is prevented and improved;
(16) A method for preventing and improving fatigue, characterized in that a health food containing proanthocyanidin as an effective ingredient is administered to human being;
(17) The method for preventing and improving fatigue according to the above (16), wherein the health food is a solid food, a gel-formed food or a beverage;
(18) The method for preventing and improving fatigue according to the above (17), wherein the beverage is a refreshing beverage or a tea beverage;
(19) The method for improving physiological motor functions according to the above (13), wherein there is used a form of tablet, pill, capsule, granule, powder, diluted powder or liquid;
(20) The method for preventing and improving fatigue according to the above (16), wherein there is used a form of tablet, pill, capsule, granule, powder, diluted powder or liquid;
(21) The method for improving physiological motor functions according to the above(13), wherein proanthocyanidin is an extract derived from pine bark;
(22) The method for improving physiological motor functions according to the above (13), wherein proanthocyanidin is an oligomeric proanthocyanidin;
(23) The method for preventing and improving fatigue according to the above (16), wherein proanthocyanidin is an extract derived from pine bark; and
(24) The method for preventing and improving fatigue according to the above (16), wherein proanthocyanidin is an oligomeric proanthocyanidin.
   The present invention still further relates to the followings.
(25) A use of proanthocyanidin for the manufacture of a medicine which improves physiological motor functions;
(26) The use according to the above (25), wherein fatigue is prevented and improved;
(27) The use according to the above (25), wherein muscular fatigue is prevented and improved;
(28) A use of proanthocyanidin for the manufacture of a health food for prevention and improvement of fatigue;
(29) The use according to the above (28), wherein the health food is a solid food, a gel-formed food or a beverage;
(30) The use according to the above (29), wherein the beverage is a refreshing beverage or a tea beverage;
(31) The use according to the above (25), wherein there is used a form of tablet, pill, capsule, granule, powder, diluted powder or liquid;
(32) The use according to the above (28), wherein there is used a form of tablet, pill, capsule, granule, powder, diluted powder or liquid;
(33) The use according to the above (25), wherein proanthocyanidin is an extract derived from pine bark;
(34) The use according to the above (25), wherein proanthocyanidin is an oligomeric proanthocyanidin;
(35) The use according to the above (28), wherein proanthocyanidin is an extract derived from bark of pine; and
(36) The use according to the above (28), wherein proanthocyanidin is an oligomeric proanthocyanidin.

### EFFECT OF THE INVENTION

The composition of the present invention can suppress the rise in lactic acid value upon physical exercise and make continuous and effective physical exercise easy. Especially when new physical exercise is started, load to muscle tissues suddenly increases locally and, therefore, feeling of fatigue increases and that is apt to disturb the continued physical exercise. The composition of the present invention has an advantage that such a disorder is suppressed and physiological motor functions are enhanced whereupon a continuous physical exercise can be easily carried out.

### BEST MODE FOR CARRYING OUT THE INVENTION

Proanthocyanidin used in the present invention means a group of compounds, derivatives and stereoisomers thereof comprising condensation polymers having a degree of polymerization of not less than 2, preferably dimer to decamer and, more preferably, dimer to tetramer where flavan-3-ol and/or flavane-3,4-diol are/is constituting unit(s). Among the proanthocyanidin, a condensation polymer having a degree of polymerization of 2 to 4 where flavan-3-ol and/or flavane-3,4-diol are/is constituting unit(s) is called OPC (oligomeric proanthocyanidin). OPC is a powerful antioxidant substance (refer to Japanese Patent Publication 3-7232 B) and is abundantly contained in leaves and barks of plants and rinds or seeds of fruits. To be more specific, it is contained in grape, pine bark, inner skin of peanut, ginkgo, fruit of false acacia (Robinia pseudoacacia), cowberry (Vaccinium vitisidaea), blueberry, strawberry, avocado, barley, wheat, soybean, black soybean, cacao, etc. It has been also known that OPC is contained in cola nut of West Africa and root of ratany of Peru. OPC is a substance which is not produced in human body.

With regard to proanthocyanidin contained in the agent for improving physiological motor functions, etc. of the present invention, there is no limitation at all for the source of the material or utilizing part, manufacturing method, purifying method, etc. of the material but there may be used a food material such as a crushed product of the above-mentioned bark, fruit or seed or an extract thereof. It is preferred to use an extract of pine bark or, more preferably, pine bark produced in the seaside of France in which OPC is abundantly contained. Bark of pine produced at the seaside of France is preferably used as a material for proanthocyanidin.

Proanthocyanidin can be easily prepared from the above-mentioned various plants by adopting a known method (such as a method mentioned in Japanese Patent Publication 3-7232 B or an extracting method from pine bark (R. W. Hemingway, et al. , Phytochemistry, 1983, Volume 22, pages 275 to 281)) or a method similar thereto.

Proanthocyanidin prepared as above is in a form of liquid or semi-solid and, when an extracting solvent is removed therefrom by a known method such as evaporation in vacuo, spray drying or freeze drying, the product per se may be used as a proanthocyanidin-containing concentrate or dry substance. For further purification, an object can be achieved by adoption of a known purifying means such as column chromatography and countercurrent distribution.

Proanthocyanidin in the composition of the present invention such as an agent for improving physiological motor functions is well soluble in water and is highly absorbed into living body. Its stability is high under all conditions of acidic, neutral and alkaline and it is easy to compound with food/beverage under the state of maintaining its function. In addition, its effect can be expected within short time after its ingestion and a sufficient effect is achieved even by ingestion of small amount and, therefore, its utility is high as a food material to small children and aged people where allowable dose for ingestion and ingesting form as food/beverage are limited, and also as a packed meal for continued administration during training at a camp of sports club members.

The composition of the present invention such as an agent for improving physiological motor functions may be a composition for any use for the improvement of physiological motor functions and, for example, it may be food/beverage, health food, functional food and pharmaceutical composition. In any of those uses, the composition may be appropriately made into a solid or liquid form such as tablet, pill, capsule, granule, powder, diluted powder and solution by publicly known methods. Thus, a solid preparation or a liquid preparation of the present invention which is useful as an agent for improving physiological motor functions, etc. is manufactured by mixing proanthocyanidin with various additives if necessary followed by using known methods for preparations which have been fully established already. For example, the above-mentioned compositions may be used, for example, in admixture with excipients, pH adjusting agents, refreshing agents, suspending agents, diluting agents, anti-foaming agents, thickening agents, solubilizers, disintegrating agents, binders, lubricants, antioxidants, coating agents, coloring agents, agents for masking taste and/or smell, surfactants, plasticizers or perfumes.

Examples of the above-mentioned excipients are sugar alcohol such as D-sorbitol, D-mannitol and xylitol, saccharide such as glucose, sucrose, lactose and fructose, crystalline cellulose, carmellose sodium, calcium hydrogen phosphate, wheat starch, rice starch, corn starch, potato starch, dextrin, β-cyclodextrin, light silicic acid anhydride, titanium oxide and magnesium aluminium metasilicate.

Examples of the above-mentioned pH adjusting agents are citric acid, malic acid, sodium hydrogen phosphate and dipotassium hydrogen phosphate.

Examples of the above-mentioned refreshing agents are 1-menthol and peppermint solution.

Examples of the above-mentioned suspending agents are kaolin, carmellose sodium, xanthan gum, methylcellulose and tragacanth.

Examples of the above-mentioned diluting agents are pure water, ethanol, plant oil and emulsifier.

Examples of the above-mentioned antifoaming agents are dimethyl polysiloxane and silicon antifoaming agent.

Examples of the above-mentioned thickeners are xanthan gum, tragacanth, methylcellulose and dextrin.

Examples of the above-mentioned solubilizers are ethanol, sucrose fatty acid ester and Macrogol.

Examples of the above-mentioned disintegrating agents are low-substituted hydroxypropyl cellulose, carboxymethyl cellulose calcium, croscarmellose sodium, hydroxypropyl starch and α-starch.

Examples of the above-mentioned binders are methyl cellulose, hydroxypropyl cellulose, hydroxypropylmethyl cellulose, polyvinylpyrrolidone, gelatin, gum arabic ethylcellulose, polyvinyl alcohol, pullulan, α-starch, agar, tragacanth, sodium alginate and propylene glycol alginate.

Examples of the above-mentioned lubricants are stearic acid, magnesium stearate, calcium stearate, polyoxyl stearate, cetanol, talc, hydrogenated oil, sucrose fatty acid ester, dimethyl polysiloxane, beeswax and bleached beeswax.

Examples of the above-mentioned antioxidants are dibutylhydroxytoluene (BHT), propyl gallate, butylhydroxyanisole (BHA), tocopherol, ascorbic acid and citric acid.

Examples of the above-mentioned coating agents are hydrodxypropyl methylcellulose, hydroxypropyl cellulose, methylcellulose, ethylcellulose, hydroxypropyl methylcellulose phthalate, hydroxypropyl methylcellulose acetate succinate, carboxymethylethyl cellulose, cellulose acetate phthalate, polyvinyl acetal diethylaminoacetate, aminoalkyl methacrylate copolymer, hydroxypropylmethyl cellulose acetate succinate, methacrylic acid copolymer, polyvinylacetate diethylaminoacetate and shellac.

Examples of the above-mentioned coloring agents are extract of turmeric (Curcuma longa), riboflavin, titanium oxide and carotene solution.

Examples of the above-mentioned masking agents for taste and/or smell are fructose, D-sorbitol, glucose, saccharin sodium, simple syrup, sucrose, honeybee, sweet hydrangea, licorice root, citric acid, adipic acid, ascorbic acid, orange oil, tincture of dried orange peel, fennel oil, peppermint and menthol.

Examples of the above-mentioned surfactants are polyoxyethylene hydrogenated castor oil, glycerol monostearate, sorbitan monostearate, sorbitan monolaurate, polyoxyethylene polyoxypropylene, polysorbate, sodium lauryl sulphate, Macrogol and sucrose fatty acid ester.

Examples of the above-mentioned plasticizers are triethyl citrate, polyethylene glycol, triacetin and cetanol.

Examples of the above-mentioned perfumes are natural perfume such as animal perfume or plant perfume; isolated perfume; and synthetic perfume such as totally synthesized perfume.

Amount of proanthocyanidin in the preparation is usually about 1 to 80% by weight or, preferably, about 2 to 40% by weight to the whole preparation.

In the case of food/beverage, proanthocyanidin or the above-mentioned proanthocyanidin-containing preparation is compounded during the manufacture of the food/beverage. For example, it is possible to make into various food forms including solid food such as bread, chewing gum, cookie, chocolate and cereal, food in a form of jam, cream or gel such as jam, ice cream, yogurt and jelly and beverage such as juice, coffee, cocoa, green tea, oolong tea and tea. It is also possible to compound with seasonings, food additives, etc.

Although the dose of the agent for improving physiological motor functions according to the present invention varies depending upon its type and dosage form and also upon age, body weight, symptom to be applied or state of a patient or a person who ingests it, it is recommended in the case of, for example, an oral preparation, to administer about 1 to 500 mg or, preferably, about 3 to 200 mg at a time for several times a day to an adult. When it is ingested as a health food, there is no problem even if the same dose as in the case of oral preparation is ingested because there is no anxiety of side effect. Although the ingesting timing is not particularly set out, higher effect can be expected particularly when it is ingested within one hour before and after the physical exercise.

Hereinafter, the present invention will be illustrated in detail by taking an extract of pine bark of French seaside containing abundant OPC as an example although the present invention is not limited thereto.

### EXAMPLE 1

Manufacture of Tablets

There were prepared tablets (each tablet weighing 250 mg) in which 8% by weight of an extract of pine bark of French seaside (containing not less than 40% by weight of proanthocyanidin (not less than 20% by weight as an OPC) and not less than 5% by weight of catechin), 22% by weight of crystalline cellulose, 66% by weight of lactose, 3% by weight of sucrose ester and 1% by weight of silicon dioxide were compounded (a test food).

### COMPARATIVE EXAMPLE

As a placebo food, a product where crystalline cellulose was compounded instead of the extract of pine bark of French seaside was manufactured (a comparative food).

### EXAMPLE 2

FRAP activity test and antioxidant evaluation test using rats

Male rats of Wistar strain (9 weeks age) were fasted for one night, a pine bark extract mentioned in Example 1 or vitamin C was compulsorily administered via oral route, and blood was collected from tail vein from time to time to prepare plasma samples. FRAP value of the plasma sample was measured by a method of Benzie (Benzie, I. F. F. and Strain, J. J., Anal. Biochem. , 1996, Volume 239, pages 70 to 76) and its changes with a lapse of time were traced as an index for antioxidant activity in blood.

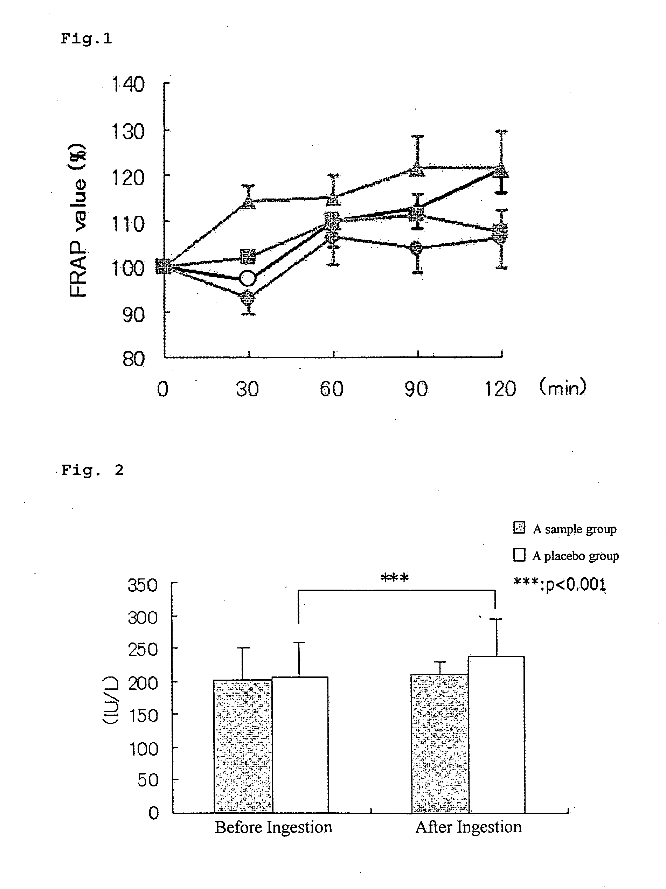

The plasma sample (10 µL) was added to 990 µL of FRAP reagent containing 20 mM of FeCl₃ followed by being allowed to stand at 37°C for 4 minutes, and changes in absorbance at 593 nm were measured. The same operation was conducted using a solution containing a predetermined concentration of FeCl₃ as a standard sample and, from the changes in absorbance, a calibration curve for FRAP values was prepared. As a result, it was found that, when the pine bark extract was 20 mg/kg or more, the FRAP value in blood increased after 60 to 120 minutes from the administration and, when the pine bark extract was 100 mg/kg, the FRAP values increased to about 10% and about 20% after 30 to 60 minutes and 90 to 120 minutes from the administration, respectively (Fig. 1). Incidentally, an increase in the FRAP value was also observed by administration of 100 mg/kg of vitamin C, and when the concentration was same, administration of the pine bark extract gave quicker increase in the FRAP value.

### EXAMPLE 3

Effect to physical exercise training of aged people

An up and down exercise training using a stepping stand was carried out for one week by 26 test subjects of 70 to 86 years age. The subjects were divided into two groups of a group for ingesting the sample and another group for ingesting a placebo and, from the starting day of the training, two tablets of the tablets mentioned in Example 1 (test food) were ingested per day to the sample group while two tablets of the comparative food mentioned in Comparative Example were ingested per day to the placebo group. Blood was collected from the subjects on the starting day and the final day of the training and, as an index for the fatigue, changes in lactate dehydrogenase (LDH) in plasma in the sample group and in the placebo group were compared. As a result, a significant rise in LDH was observed in the placebo group while, in the sample group, a rise in LDH was suppressed (Fig. 2).

### EXAMPLE 4

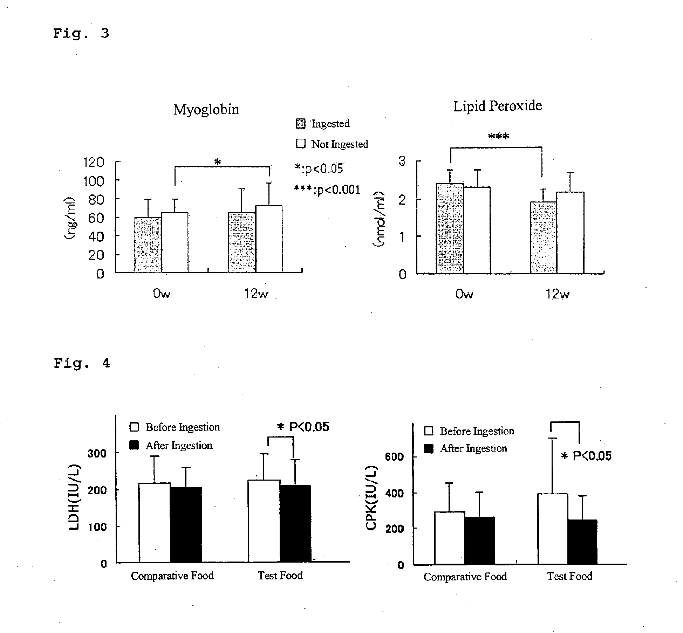

An up-and-down exercise training using a stepping stand was carried out for 12 weeks by 25 test subjects of 70 or older age. The subjects were divided into two groups of a group for ingesting the sample and another group for ingesting nothing and, from the starting day of the training, two tablets of the tablets mentioned in Example 1 (test food) were ingested per day to the sample group. Blood was collected from the subjects on the starting day and the final day of the training and, as an index for muscular fatigue, myoglobin and lipid peroxide in serum of each group were measured. As a result, it was confirmed that, by ingestion of the sample, a rise in myoglobin was suppressed and lipid peroxide lowered. (Fig. 3).

### EXAMPLE 5

Effect on physiological motor functions in a training of long-distance runners

A double blind crossover test was carried out in such a manner that each 4 tablets of the tablets mentioned in Example 1 (test food) and the comparative food mentioned in Comparative Example as a placebo were administered per day for seven days to 12 male long-distance runners as subjects to be tested. The subjects were subjected to the same training during the period for ingesting the test food and the period for ingesting the comparative food for seven days each and, before and after those periods, blood tests were carried out.

As an index for the fatigue, lactate dehydrogenase (LDH) and creatinine kinase (CPK) in plasma were measured by means of a biochemical blood test and, as a result, neither LDH nor CPK changed before and after the periods for ingestion of the comparative food. On the other hand, after completion of the period for ingestion of the test food, both LDH and CPK significantly lowered as compared with the stage before the ingestion (Fig. 4).

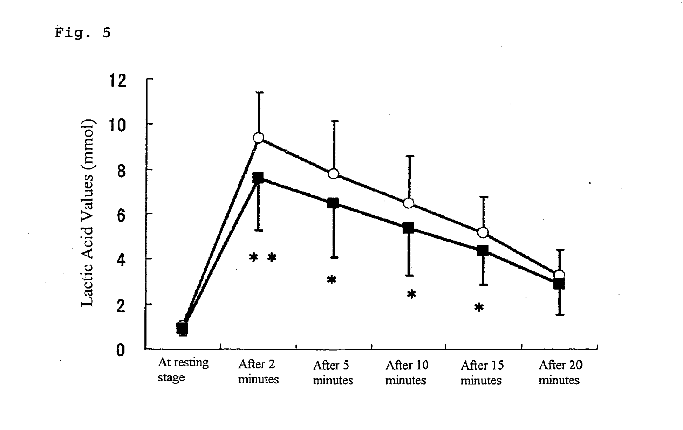

In addition, as an index for muscular fatigue, the maximum overload exercise was conducted on the last day of each period and lactic acid concentrations in blood before and after that were periodically measured. The result was that, with regard to the lactic acid value in blood at the rest stage before an exercise load, there was no difference between the two periods while, from after 2 to 15 minutes from the exercise where a rise in lactic acid value was observed, significantly low values were observed in the ingestion of the test food as compared with the ingestion of the comparative food (Fig. 5).

During the test period, the test food and the comparative food were given to each of the test subjects on a blind base by a crossover means and the test was carried out in such a manner that even the measuring person was not advised which tablet was ingested by each test subject. Still, both test subjects and measuring person could correctly make a good guess for the period of being ingested with the test food.

### INDUSTRIAL APPLICABILITY

The composition of the present invention is useful as an agent for preventing and improving the fatigue in which the rise in lactic acid value upon physical exercise is suppressed whereby muscular fatigue is reduced and continuous exercise is made easy, and particularly as an agent for prevention and improvement of muscular fatigue, and as a health food for prevention and improvement of fatigue.

### BRIEF DESCRIPTION OF THE DRAWINGS

Fig. 1 shows periodical changes of FRAP values in rats to which the pine bark extract was administered. In the drawing, ● is a group to which 20 mg/kg of the pine bark extract was administered, ■ is a group to which 50 mg/kg of the pine bark extract was administered, ▲ is a group to which 100 mg/kg of the pine bark extract was administered and ○ is a group to which 100 mg/kg of vitamin C was administered.
Fig. 2 shows an influence of ingestion of the pine bark extract on a rise of lactate dehydrogenase in plasma upon a training for one week.
Fig. 3 shows an influence of ingestion of the pine bark extract on rises of myoglobin and lipid peroxide in serum upon a training for 12 weeks.
Fig. 4 shows an influence of ingestion of the pine bark extract on lactate dehydrogenase (LDH) and creatinine kinase (CPK) in plasma in long-distance runners.
Fig. 5 shows an influence of ingestion of the pine bark extract on a rise of lactic acid value in blood in long-distance runners. An abscissa shows elapse of time after starting the exercise load. In the drawing, ■ is a group ingesting the test food and ○ is a group ingesting the comparative food. * shows a statistically significant difference p < 0.05 for the group ingesting the comparative food and ** shows a statistically significant difference p < 0.01 for the group ingesting the comparative food.

## Claims

1. An agent for improving physiological motor functions which is **characterized in** containing proanthocyanidin as an effective ingredient.

2. The agent for improving physiological motor functions according to claim 1, wherein fatigue is prevented and improved.

3. The agent for improving physiological motor functions according claim 1, wherein muscular fatigue is prevented and improved.

4. A health food for prevention and improvement of fatigue which is **characterized in** containing proanthocyanidin as an effective ingredient.

5. The health food for prevention and improvement of fatigue according to claim 4, wherein the health food is a solid food, a gel-formed food or a beverage.

6. The health food for prevention and improvement of fatigue according to claim 5, wherein the beverage is a refreshing beverage or a tea beverage.

7. The agent for improving physiological motor functions according to claim 1, wherein the agent is used in the form of tablet, pill, capsule, granule, powder, diluted powder or liquid.

8. The health food for prevention and improvement of fatigue according to claim 4, wherein the health food is used in the form of tablet, pill, capsule, granule, powder, diluted powder or liquid.

9. The agent for improving physiological motor functions according to claim 1, wherein proanthocyanidin is an extract derived from pine bark.

10. The agent for improving physiological motor functions according to claim 1, wherein proanthocyanidin is an oligomeric proanthocyanidin.

11. The health food for prevention and improvement of fatigue according to claim 4, wherein proanthocyanidin is an extract derived from pine bark.

12. The health food for prevention and improvement of fatigue according to claim 4, wherein proanthocyanidin is an oligomeric proanthocyanidin.

13. A method for improving physiological motor functions, **characterized in that** proanthocyanidin is administered to human being.

14. The method for improving physiological motor functions according to claim 13, wherein fatigue is prevented and improved.

15. The method for improving physiological motor functions according to claim 13, wherein muscular fatigue is prevented and improved.

16. A method for preventing and improving fatigue, **characterized in that** a health food containing proanthocyanidin as an effective ingredient is administered to human being.

17. The method for preventing and improving fatigue according to claim 16, wherein the health food is a solid food, a gel-formed food or a beverage.

18. The method for preventing and improving fatigue according to claim 17, wherein the beverage is a refreshing beverage or a tea beverage.

19. The method for improving physiological motor functions according claim 13, wherein there is used a form of tablet, pill, capsule, granule, powder, diluted powder or liquid.

20. The method for preventing and improving fatigue according to claim 16, wherein there is used a form of tablet, pill, capsule, granule, powder, diluted powder or liquid.

21. The method for improving physiological motor functions according to claim 13, wherein proanthocyanidin is an extract derived from pine bark.

22. The method for improving physiological motor functions according to claim 13, wherein proanthocyanidin is an oligomeric proanthocyanidin.

23. The method for preventing and improving fatigue according to claim 16, wherein proanthocyanidin is an extract derived from pine bark.

24. The method for preventing and improving fatigue according to claim 16, wherein proanthocyanidin is an oligomeric proanthocyanidin.

25. A use of proanthocyanidin for the manufacture of a medicine which improves physiological motor functions.

26. The use according to claim 25, wherein fatigue is prevented and improved.

27. The use according to claim 25, wherein muscular fatigue is prevented and improved.

28. A use of proanthocyanidin for the manufacture of a health food for prevention and improvement of fatigue.

29. The use according to claim 28, wherein the health food is a solid food, a gel-formed food or a beverage.

30. The use according to claim 29, wherein the beverage is a refreshing beverage or a tea beverage.

31. The use according to claim 25, wherein there is used a form of tablet, pill, capsule, granule, powder, diluted powder or liquid.

32. The use according to claim 28, wherein there is used a form of tablet, pill, capsule, granule, powder, diluted powder or liquid.

33. The use according to claim 25, wherein proanthocyanidin is an extract derived from pine bark.

34. The use according to claim 25, wherein proanthocyanidin is an oligomeric proanthocyanidin.

35. The use according claim 28, wherein proanthocyanidin is an extract derived from pine bark.

36. The use according to claim 28, wherein proanthocyanidin is an oligomeric proanthocyanidin.
